# EUROPÄISCHE PATENTSCHRIFT

(11) **EP 0 769 296 B1**
(45) Veröffentlichungstag und Bekanntmachung des Hinweises auf die Patenterteilung: **04.07.2001**
(21) Anmeldenummer: 96116408.4
(22) Anmeldetag: 14.10.1996
(51) Int. Cl.: A61K 31/42, A61K 31/275, A61K 31/44

(54) **Verwendung eines Natrium- oder Lysiniumsalzes von Crotonsäureamidderivaten zur Herstellung eines Arzneimittels zur Behandlung von Krebserkrankungen**
Use of a sodium or lysin salt of crotonamide derivatives for the manufacture of a medicament for the treatment of cancers
Utilisation d'un sel de sodium ou de lysine des derivés de crotonamide pour la fabrication d'un médicament pour le traitement des cancers

(30) Priorität: 25.10.1995 DE 19539638
(43) Veröffentlichungstag der Anmeldung: 23.04.1997
(73) Patentinhaber: Aventis Pharma Deutschland GmbH, 65929 Frankfurt am Main (DE)
(72) Erfinder: Schwab, Wilfried, Dr., 65207 Wiesbaden (DE); Czech, Jörg, Dr., 35041 Marburg (DE); Boslett, Klaus, Dr., 35037 Marburg (DE)

(56) Entgegenhaltungen:
- EP-A- 0 646 578
- EP-A- 0 665 013
- WO-A-91/17748
- WO-A-95/19169

## Beschreibung

Für die Therapie fortgeschrittener Hormonrezeptor negativer maligner Tumore steht heutzutage die Chemotherapie zur Verfügung. Diese Therapieform ist neben ihrer begrenzten Effizienz durch das Auftreten oft schwerwiegender Nebenwirkungen gekennzeichnet. Als Ursache für die Wirkung und die Nebenwirkungen, muß das Wirkprinzip der Chemotherapeutika (die Proliferationsinhibition) angesehen werden. Da sich aber nicht nur Tumorzellen, sondern auch normale Zellen in Teilung befinden, werden normale sich teilende Zellen im Körper des Patienten ebenso an der Teilung gehemmt wie die eigentlichen Zielzellen, die Tumorzellen. Besonders betroffen sind von den unerwünschten Nebenwirkungen der antiproliferativen Therapie, die sich schnell teilenden Zellen der Haarfollikel, des Gastrointestinaltraktes und des Knochenmarkes.

Die antiproliferative Wirkung der Chemotherapeutika wird zum Beispiel dadurch erreicht, daß sie in den Nukleinsäurestoffwechsel der Zelle eingreift. Besonders wirkungsvolle antiproliferative Stoffe sind die Dihydroorotat-dehydrogenase (DHODH)-Hemmer. Die DHODH ist ein einzigartiges Enzym in der de novo-Synthese der Pyrimidinnukleotide (Peters et al., 1990, Biochemical Pharmacology 39: Nr. 1, 135-144). Das Enzym ist auf der Außenseite der inneren Mitochondrienmembran gelegen. Hemmung des Enzyms durch den Wirkstoff DUP-785 (Brequinar) führt zu einer Depletion von Pyrimidinribo- und Desoxyribonukleotiden, nicht aber von Purinnukleotiden (Schwartsmann et al., 1988, Biochem. Pharmcol. 37: 3257-3266). Die Depletion von dTTP und dCTP ist derjenigen von UTP und CTP proportional und kann durch die Zugabe von Uridin verhindert werden. Die durch Brequinar auf in vitro Zellinien ausgeübte Wachstumshemmung kann durch Zugabe von Uridin oder Cytidin aufgehoben werden, nicht aber durch Desoxythymidin oder Desoxycytidin. Hieraus kann gefolgert werden, daß die Hemmung der UMP-Synthese für den Proliferationshemmenden Effekt auf Zellinien in vitro entscheidend ist (Peters et al., 1987, Invest. New Drugs, 5: 235-244). WO 95/19169 beschreibt Crotonsäureamide der allgemeinen Formel II, sowie deren Verwendung zur Behandlung von Krebserkrankungen.

Im Rahmen klinischer Untersuchungen konnte gezeigt werden, daß Brequinar die Plasmauridinwerte signifikant absenkt (Peters er al., 1988, Proc. Am Ass. Cancer Res. 29: 350 (Abstract 1392)) (dieser Befund ist in Übereinstimmung mit den in vitro Beobachtungen auf verschiedenen Zellinien). Des weiteren korrelierte das Ausmaß an Effekten auf die in vivo-Uridinspiegel mit der Knochenmarks- und Gastrointestinaltrakt-Toxizität (den Nebenwirkungen).

Diese klinischen Beobachtungen deuten darauf hin, daß die dringende Notwendigkeit besteht, Krebstherapeutika zu entwickeln, deren anti-tumorales Prinzip nicht auf einer allgemeinen Proliferationsinhibition, wie beim Brequinar, sondern auf der Hemmung tumorspezifischer Stoffwechselwege beruht.

Es wurde nun gefunden, daß die Verbindungen der Formeln I und II die humane DHODH nur sehr schwach hemmen, jedoch ganz bestimmte Tumorzellinien sehr effizient in ihrer Teilung blockieren.

Zugabe steigender Mengen von Uridin in den MTT-Test verändert den IC₅₀-Wert der erfindungsgemäßen Substanzen auf der Lo-Vo-Zellinie nur unwesentlich, ganz im Gegensatz zur massiven Erhöhung des IC₅₀-Wertes von Brequinar auf der gleichen Zellinie (Beispiel 7).

Diese experimentellen Befunde unterstützen die Annahme, daß die antiproliferative Wirkung der erfindungsgemäßen Substanzen auf einem anderen Wirkprinzip als einer DHODH-Inhibition wie beim Brequinar beruht.

Mittels fluoreszens-mikroskopischer Techniken konnte gezeigt werden, daß diejenigen Zellinien, welche den PDGF-Rezeptor und den VEGF-Rezeptor stark überexprimieren auch durch sehr niedrige Konzentrationen der erfindungsgemäßen Substanzen (niedriger IC₅₀) effizient an der Teilung gehindert werden (Beispiel 6). Diese Beobachtung deutet daraufhin, daß die erfindungsgemäßen Substanzen bestimmte Rezeptortyrosinkinasen, wie z.B. den PDGF-Rezeptor blockieren könnten, d.h. die anormale Signaltranstuktion bei Tumorzellen vorteilhaft beeinflussen.

Die Erfindung betrifft daher die Verwendung eines Natrium- oder Lysiniumsalzes der Verbindung der Formel II 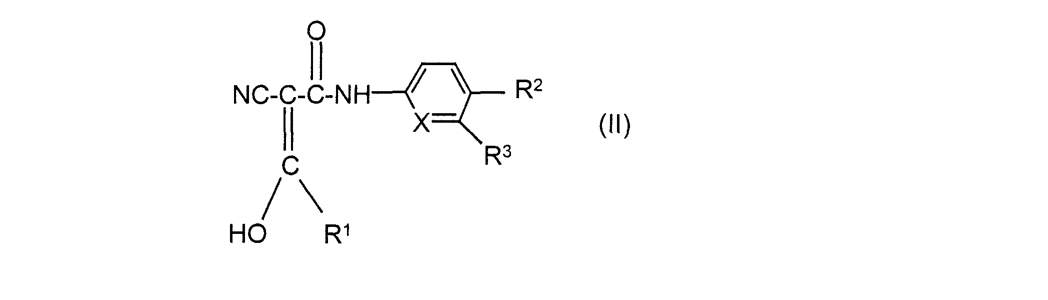 und/oder eine gegebenenfalls stereoisomere Form des Natrium- oder Lysiniumsalzes der Verbindung der Formel II zur Herstellung eines Arzneimittels zur Behandlung von Krebserkrankungen, wobei
- R¹ für: a) (C₃-C₅)-Cycloalkyl,
b) (C₂-C₆)-Alkenyl oder
c) (C₂-C₆)-Alkinyl, steht,
- R² für: a) -CF₃,
b) -O-CF₃,
c) -S-CF₃,
d) -OH,
e) -NO₂,
f) Halogen,
g) Benzyl,
h) Phenyl,
i) -CN,
k) -O-Phenyl,
l) -O-Phenyl, ein oder mehrfach substituiert durch
   1) (C₁-C₄)-Alkyl,
   2) Halogen,
   3) -O-CF₃ oder
   4) -O-CH₃, steht,
- R³ für: a) (C₁-C₄)-Alkyl,
b) Halogen, oder
c) Wasserstoffatom steht, und
- X für: a) eine -CH-Gruppe oder
b) Stickstoffatom, steht.

Bevorzugt ist die Verwendung eines Natrium- oder Lysiniumsalze der Verbindung der Formel II, wobei
- R¹ für: Cyclopropyl, (C₂-C₃)-Alkenyl oder (C₃-C₅)-Alkinyl steht,
- R² für: -O-CF₃, -S-CF₃, -O-Phenyl, Phenyl, -CF₃, -CN oder -O-Phenyl, ein oder mehrfach substituiert durch (C₁-C₄)-Alkyl oder Halogen, steht,
- R³ für: Wasserstoffatom oder Methyl steht, und
- X für: eine -CH-Gruppe steht,
zur Herstellung eines Arzneimittels zur Behandlung von Krebserkrankungen.

Besonders bevorzugt ist die Verwendung eines Natrium- oder Lysinium salzes der Verbindung der Formel II, wobei
- R¹ für: Cyclopropyl, C₃-Alkenyl oder C₄-Alkinyl steht,
- R² für: -S-CF₃, CN, 2-Methyl-4-chlorphenyl oder CF₃ steht,
- R³ für: Wasserstoffatom steht, und
- X für: eine -CH-Gruppe steht,
zur Herstellung eines Arzneimittels zur Behandlung von Krebserkrankungen.

Insbesondere bevorzugt sind die Verwendung von N-(4-Trifluormethylphe,yl)-2-cyano-3-hydroxy-hept-2-en-6-in-carbonsäureamid-Lysin- oder Natriumsalz oder 2-Cyano-3-cyclopropyl-3-hydroxy-acrylsäure-(4-cyanophenyl)-amid-Lysin- oder Natriumsalz.

Die Herstellung der Verbindungen der Formel II erfolgt nach bekannten Verfahren wie sie in EP 13 376; EP 484 223; EP 538 783; EP 551 230 oder US 4 061 767 beschrieben werden.

Unter dem Begriff Alkyl, Alkenyl oder Alkinyl werden Reste verstanden deren Kohlenstoffkette geradkettig oder verzweigt sein kann. Ferner können die Alkenyl- oder Alkinyl- Reste auch mehrere Doppelbindungen beziehungsweise mehrere Dreifachbindungen enthalten. Cyclische Alkylreste sind beispielsweise 3- bis 5-gliedrige Monocyclen wie Cyclopropyl, Cyclobutyl oder Cyclopentyl. Die Ausgangsstoffe der chemischen Umsetzungen sind bekannt oder lassen sich nach literaturbekannten Methoden leicht herstellen. Zu den Krebserkrankungen gehören beispielsweise Leukämie, insbesondere chronische Leukämie des T- und B-Zelltyps, Hodgkin's oder non- Hodgkin's Lymphom, Karzinome, Lungenkrebs, Eierstockkrebs, Lymphknotenkrebs, Sarkome, Kaposi's Sarkom, Meningiom, Darmkrebs, Hirntumore, Brustkrebs, Magenkrebs, Pankreaskrebs, Prostatakrebs oder Hautkrebs.

Die Arzneimittel können oral, topisch, rektal, intravenös oder auch parenteral appliziert werden.

Geeignete feste oder flüssige galenische Darreichungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyethylenglykole und Lösungsmittel, wie etwas steriles Wasser und ein- oder mehrwertige Alkohole, z.B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis eines Lysin- oder Natrium salzes der Verbindung der Formel II enthält. Bei festen Dosierungseinheiten, wie Tabletten, Kapseln oder Suppositorien, kann diese Dosis bis zu etwa 300 mg, bevorzugt jedoch 10 bis 200 mg betragen.

Für die Behandlung eines Patienten (70 kg), sind in frühen Phasen eine intravenöse Infusionsbehandlung von maximal 1200 mg pro Tag und in der späteren Rehabilitationsphase eine orale Verabreichung von 3 mal 300 mg pro Tag eines Lysin- oder Natrium salzes der Verbindung der Formel II indiziert.

Unter Umständen können jedoch auch höhere oder niedrigere Dosen angebracht sein. Die Verabreichung der Dosis kann sowohl durch Einmalgabe in Form einer einzelnen Dosierungseinheit oder aber mehreren kleineren Dosierungseinheiten als auch durch Mehrfachgabe unterteilter Dosen in bestimmten Intervallen erfolgen.

Schließlich können die lysin- oder Natrium salze der Verbindungen der Formel II bei der Herstellung der vorgenannten galenischen Darreichungsformen auch -zusammen mit anderen geeigneten Wirkstoffen, beispielsweise Antiuricopathika, Thrombocytenaggregationshemmern, Analgetika und steroidalen oder nichtsteroidalen Antiphlogistika, kombiniert werden.

### Beispiel 1

### N-(4-Trifluormethylphenyl)-2-cyano-3-hydroxy-hept-2-en-6-in-carbonsäureamid Natriumsalz (Verbindung 1)

50 g (0,15 mol) N-(4-Trifluormethylphenyl)-2-cyano-3-hydroxy-hept-2-en-6-in-carbonsäureamid werden in einem Zweiphasensystem aus 50 ml 5 N Natronlauge und 500 ml Ethylacetat gelöst, die organische Phase abgetrennt, zweimal mit wenig Wasser gewaschen, über Natriumsulfat getrocknet und eingeengt. Der ölige Rückstand wird mit 500 ml tertiär-Butylmethylether aufgenommen und zur vollständigen Kristallisation 4 Stunden (h) bei Raumtemperatur gerührt, filtriert und unter vermindertem Druck getrocknet. Zur vollständigen Entfernung von Lösemittelresten wird das kristalline Produkt in 500 ml Toluol 10 min unter Rückfluß suspendiert, unter Rühren abgekühlt, erneut abgesaugt und unter vermindertem Druck getrocknet. Ausbeute: 41,1 g (77%) vom Schmelzpunkt >244°C Zersetzung (Zers.).
C₁₅H₁₀F₃N₂O₂Na (330,24 g/mol):

| | | | | | |
|---|---|---|---|---|---|
| berechnet | C: 54,0 | H: 3,5 | N:8,4 | Na: 6,88 | (ber. für 1,1% Wasser) |
| gefunden | C: 54,4 | H: 3,4 | N:8,4 | Na: 6,65 | Wasser: 1,1% |

### Beispiel 2

### 2-Cyano-3-cyclopropyl-3-hydroxy-acrylsäure-(4-cyanophenyl)-amid Natriumsalz (Verbindung 2)

15 g (0,059 mol) 2-Cyano-3-cyclopropyl-3-hydroxy-acrylsäure-(4-cyanophenyl)-amid werden in 120 ml Wasser und 100 ml Aceton suspendiert und durch Zugabe von 60 ml 1N NaOH in Lösung gebracht. Nach Filtration von Spuren an ungelöstem Material wird am Rotationsverdampfer unter vermindertem Druck auf etwa 200 ml aufkonzentriert, und bei 0°C über Nacht kristallisiert, abgesaugt und unter vermindertem Druck getrocknet.
Ausbeute: 13 g, Schmp. > 280°C.
C₁₄H₁₀N₃O₂Na (275,24):

| | | | | |
|---|---|---|---|---|
| berechnet | C: 60,7 | H: 3,7 | N:15,2 | (ber. für 0,7% Wasser) |
| gefunden | C: 60,8 | H: 3,6 | N:15,3 | Wasser: 0,7% |

### Beispiel 3

### N-(4-Trifluormethylphenyl)-2-cyano-3-hydroxy-hept-2-en-6-in-carbonsäureamid Lysinsalz

30 g (0,097 mol) N-(4-Trifluormethylphenyl)-2-cyano-3-hydroxy-hept-2-en-6-incarbonsäureamid werden zusammen mit 17,3 g (0,097 mol) L-Lysin-hydrat in 1 I Wasser und 25 ml Ethanol gelöst, filtriert, und lyophylisiert. Anhaftende Restmengen an Ethanol werden durch nochmaliges Gefriertrocknen entfernt. Ausbeute: 44,4 g an überwiegend amorphem Produkt Schmp. 135-138°C. ¹H-NMR (DMSO-d₆): 1,23-1,77 (m, 6H), 2,3-2,45 (m, 2H), 2,50-2,65 (m, 2H), 2,7-2,85 (m, 3H), 3,25 (tb, 1H), 5,7-7,4 (sb, 6H), 7,55 und 7,73 (AA'BB', jeweils 2H), 12,35 (s, 1H)

### Beispiel 4

### 2-Cyano-3-cyclopropyl-3-hydroxy-acrylsäure-(4-cyanophenyl)-amid-Lysinsalz

15 g (0,054 mol) 2-Cyano-3-cyclopropyl-3-hydroxy-acrylsäure-(4-cyanophenyl)-amid werden zusammen mit 9,6 g (0,054 mol) L-Lysin-hydrat in 900 ml Wasser und 10 ml Ethanol gelöst, filtriert, und lyophylisiert. Anhaftende Restmengen an Ethanol werden durch Trocknen unter vermindertem Druck entfernt.
Ausbeute: 21,8 g an überwiegend amorphem Produkt, Schmp. > 100°C (Zers.). ¹H-NMR (DMSO-d₆): 0,6-0,82 (m, 4H), 1,27-1,75 (m, 6H), 2,17 (mc, 1H), 2,77 (tb, 2H), 3,28 (tb, 1H), 4,8-7,5 (sb, 6H), 7,63 und 7,7 (AA'BB', jeweils 2H), 12,6 (s, 1H)

### Beispiel 5

Die humane DHODH (Milz)-Enzymaktivität wird gemäß Williamson et al. (The Journal of Biological Chemistry, 270, (1995), Seiten 22467-22472) bestimmt.

Es wird jeweils der IC₅₀-Wert in nM angegeben.

**Tabelle 1**

| **DHODH** IC₅₀ in nM | |
|---|---|
| Verbindung 1 | 292 |
| Verbindung 2 | 625 |
| Verbindung 12 | 539 |
| Brequinar | 4 |

### Beispiel 6

### Inhibition der Proliferation von Tumorzellen (MTT-Test)

In einer 96-well Mikrotiterplatte werden 1 x 10⁴ Zellen pro well ausgesät. Nach 24 h werden die Testsubstanzen in verschiedenen Konzentrationen hinzugegeben. Jede Gruppe besteht aus 4 Well, die Kontrolle wird nur mit Medium inkubiert. Nach 65 h werden 50 *µ*l MTT (3-(4,5-Dimethyl-2-thiazolyl)-2,5-diphenyl-2H-tetrazoliumbromid; 2,5 mg/ml in PBS) hinzugegeben und nach 7 h der Überstand entfernt. Der von den lebenden Zellen gebildete Farbstoff wird durch Zugabe von 100 *µ*l Dimethylsulfoxid/Well gelöst. Die Extinktion wird für jedes well mit Hilfe eines Multiscan Photometers 340 CC (Fa. Flow) bei 492 nm gemessen. Die verwendeten Zellinien sind wie folgt von der American Type Culture Collection erhältlich:
HUV-EC-C ist ATCC CRL 1730; A-172 ist ATCC CRL 1620; L 1210 ist ATCC CCL 219; LoVo ist ATCC CCL 229; C 6 ist ATCC CCL 107 und eine Rattenglioblastomzellinie.

Aus den 4 wells einer Gruppe wird der Mittelwert gebildet und aus der Dosis-Wirkungskurve die IC₅₀-Werte mit der Software 3.0 (Erithacus Software Ltd.) errechnet. Tabelle 2 zeigt die Ergebnisse.

**Tabelle 2**

| MTT-Test IC₅₀ in µM | | | | |
|---|---|---|---|---|
| Zelle | Ursprung (Human) | Verbindung 1 | Verbindung 2 | Brequinar |
| LoVo | Kolonkarzinom | 137 | 392 | 0,388 |
| HUV-EC-C | Endothelzelle | 164 | 360 | 9,4 |
| A-172 | Glioblastom | 78 | 169 | 0,2 |
| L1210 | Leukämie (Maus) | 9,1 | 6,1 | 1,0 |
| C 6 | Rattenglioblastom | 68 | 20 | 9 |

### Beispiel 7

Es wird wie in Beispiel 6 verfahren; zusätzlich wird in den Ansätzen Uridin zugefügt. Tabelle 3 zeigt die Ergebnisse.

**Tabelle 3**

| MTT-Test auf LoVo-Zellen IC₅₀ in µM | | | |
|---|---|---|---|
| Uridin [µM] | Verbindung 1 | Verbindung 2 | Brequinar |
| 0 | 168,0 | 359,2 | 0,388 |
| 1000 | 277,7 | 560,0 | 128,8 |

Die in Tabelle 4 genannten Verbindungen werden wie in den Beispielen 1 bis 4 hergestellt. Die Testung der Verbindungen erfolgt wie in Beispiel 6 beschrieben.

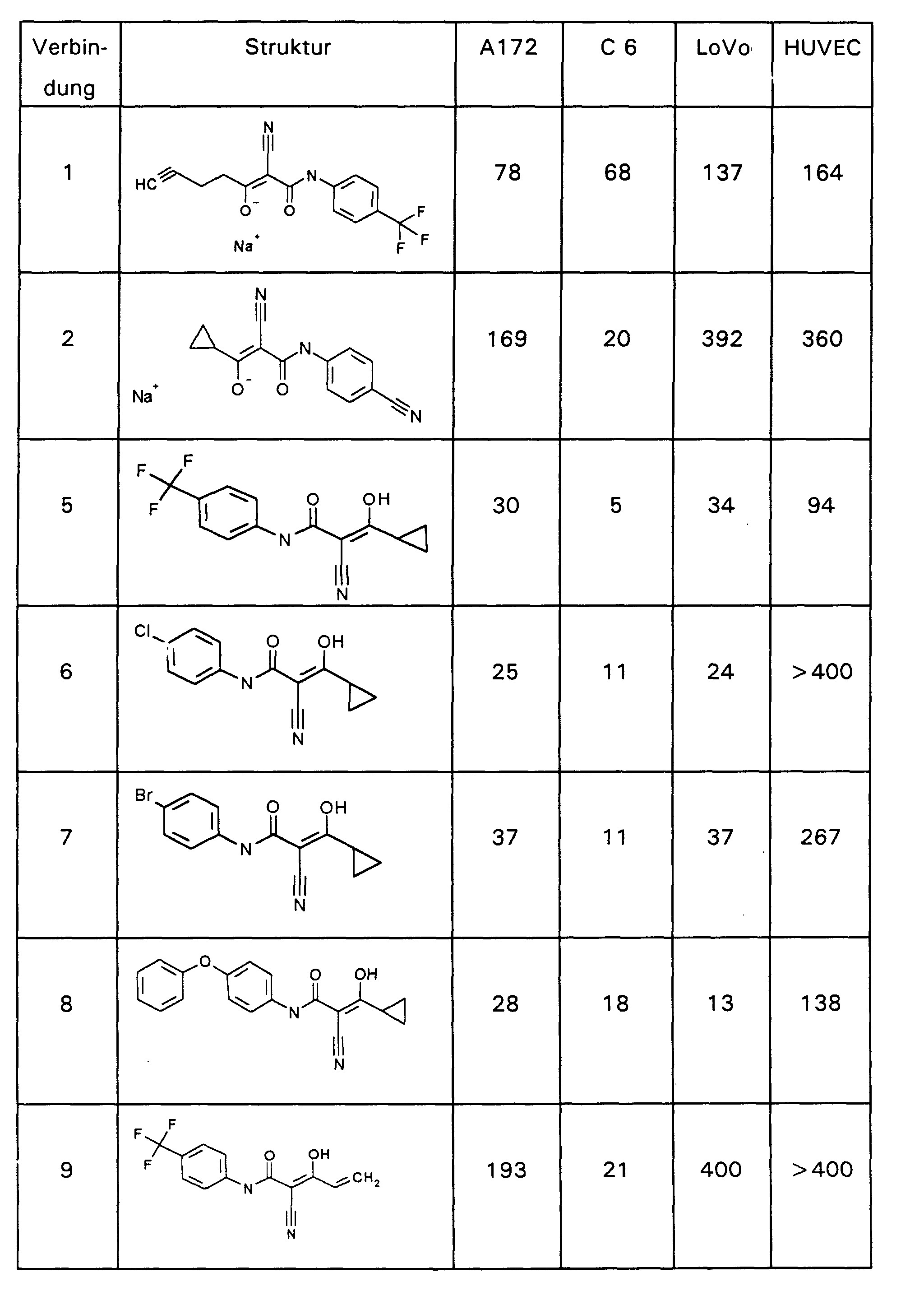

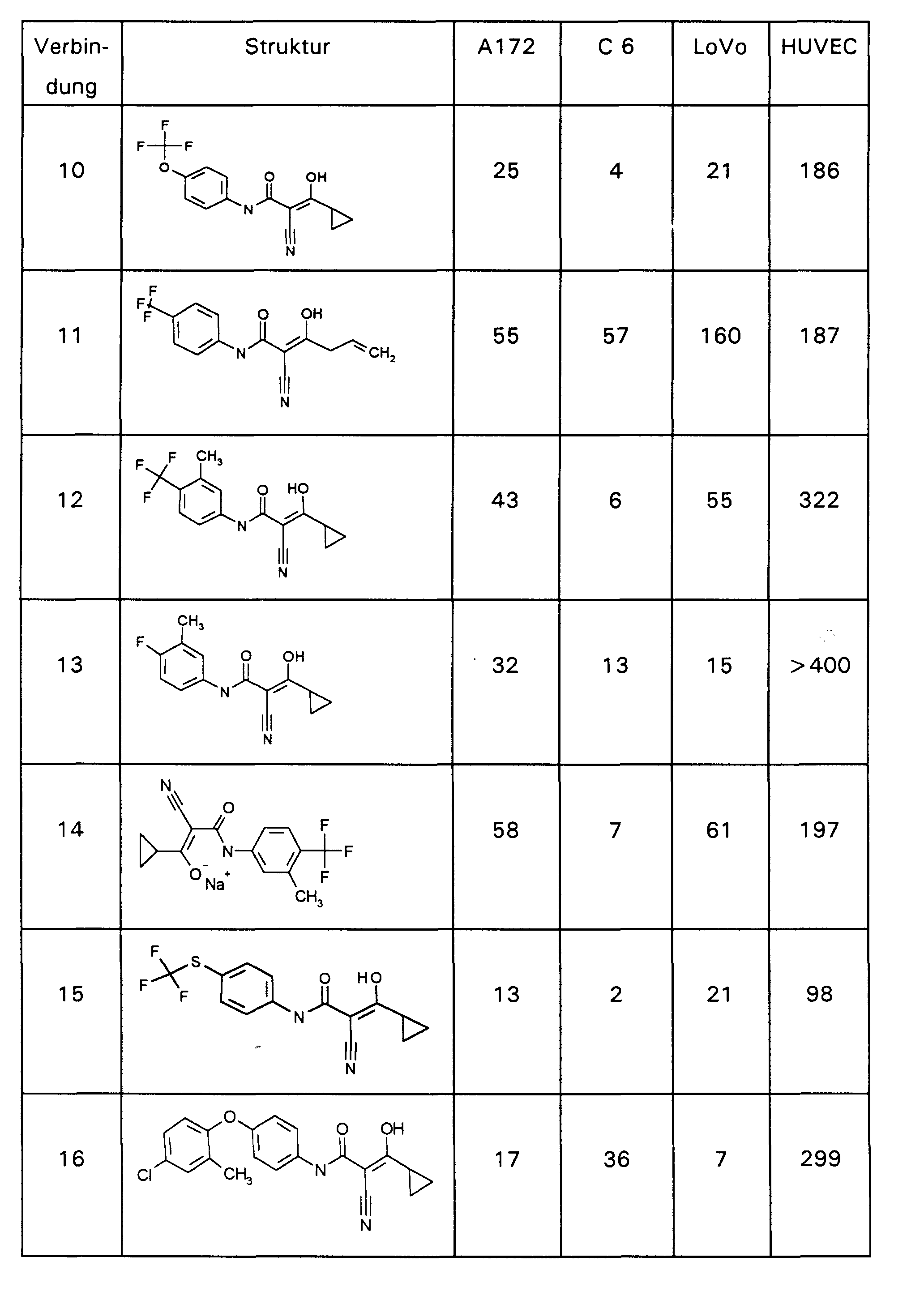

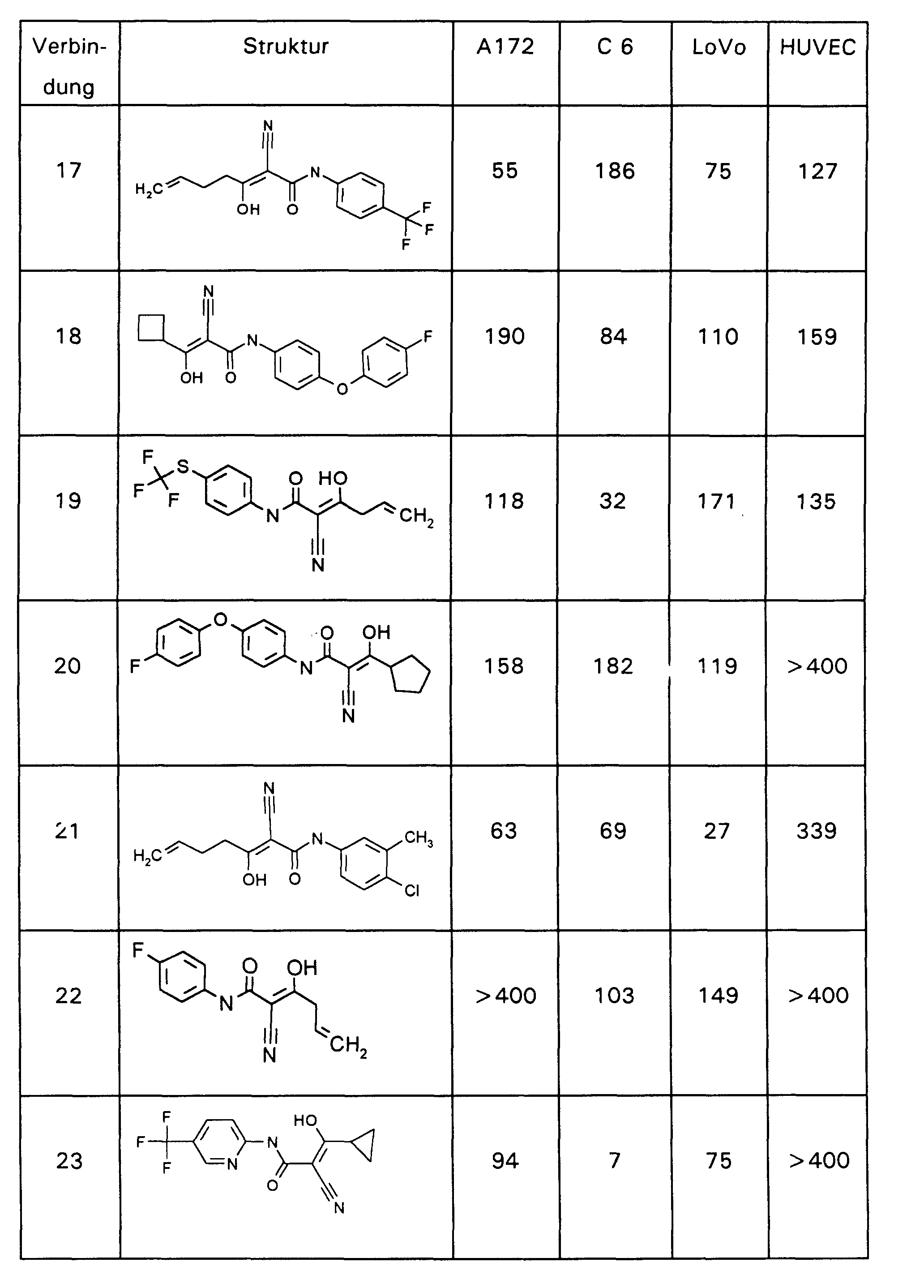

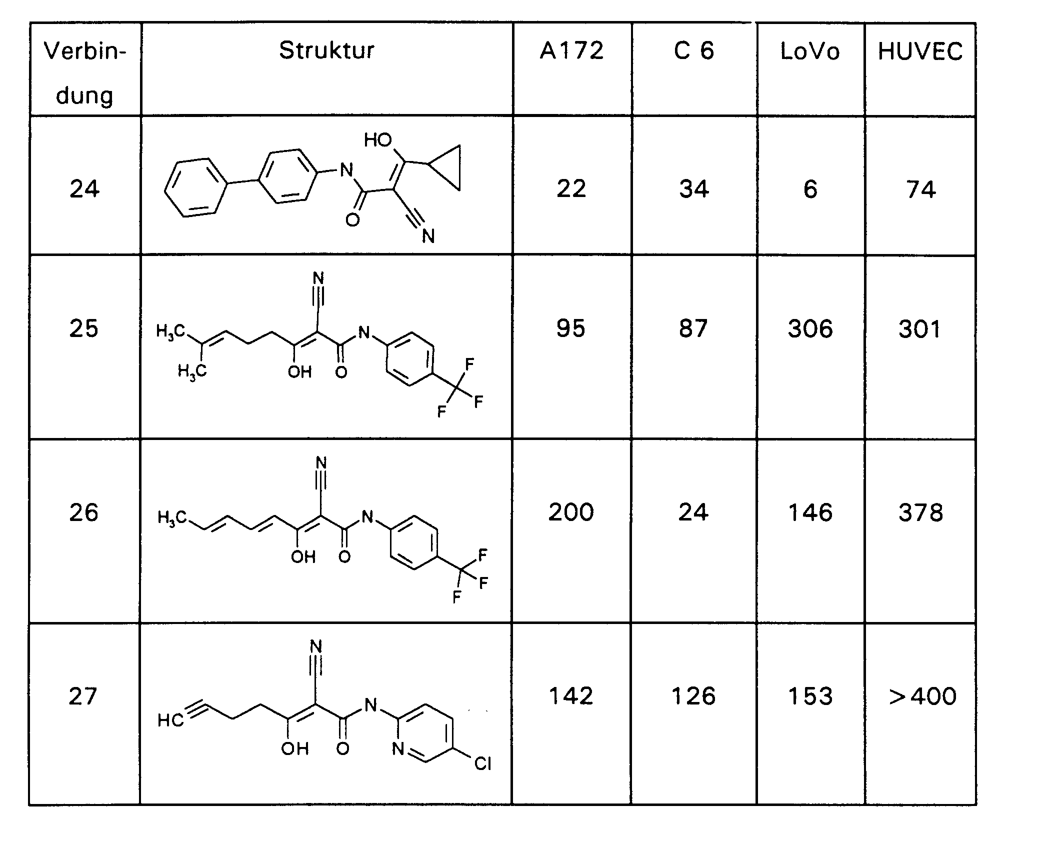

## Patentansprüche

1. Verwendung eines Natrium- oder Lysiniumsalzes der Verbindung der Formel II 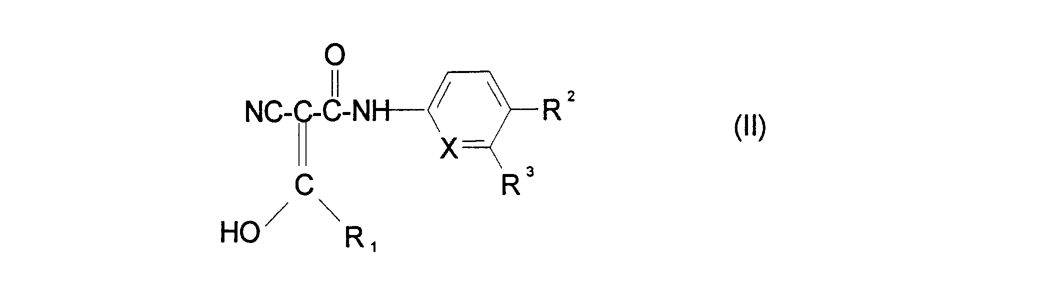 und/oder eine gegebenenfalls stereoisomere Form des Natrium- oder Lysiniumsalzes der Verbindung der Formel II zur Herstellung eines Arzneimittels zur Behandlung von Krebserkrankungen, wobei
R¹ für
a) (C₃-C₅)-Cycloalkyl,
b) (C₂-C₆)-Alkenyl oder
c) (C₂-C₆)-Alkinyl, steht,
R² für
a) -CF₃,
b) -O-CF₃,
c) -S-CF₃,
d) -OH,
e) -NO₂,
f) Halogen,
g) Benzyl,
h) Phenyl,
i) -CN,
k) -O-Phenyl oder
l) -O-Phenyl, ein oder mehrfach substituiert durch
1) (C₁-C₄)-Alkyl,
2) Halogen,
3) -O-CF₃ oder
4) -O-CH₃, steht,
R³ für
a) (C₁-C₄)-Alkyl,
b) Halogen oder
b) Wasserstoffatom steht, und
X für
a) eine -CH-Gruppe oder
b) Stickstoffatom, steht.

2. Verwendung gemäß Anspruch 1, wobei
R¹ für
a) Cyclopropyl,
b) (C₂-C₃)-Alkenyl oder
c) (C₃-C₅)-Alkinyl steht,
R² für
a) -CF3
b) -O-CF₃,
c) -S-CF₃,
d) -O-Phenyl,
e) -Phenyl,
f) -CN oder
g) -O-Phenyl, ein oder mehrfach substituiert durch (C₁-C₄)-Alkyl oder Halogen, steht,
R³ für Wasserstoffatom oder Methyl steht, und
X für eine -CH-Gruppe steht,

3. Verwendung gemäß Anspruch 1 oder 2, wobei
R¹ für Cyclopropyl, C₃-Alkenyl oder C₄-Alkinyl steht,
R² für -S-CF₃, CN, 2-Methyl-4-chlorphenyl oder CF₃ steht,
R³ für Wasserstoffatom steht, und
X für eine -CH-Gruppe steht,

4. Verwendung gemäß einer oder mehrerer der Ansprüche 1 bis 3, dadurch gekennzeichnet, daß man N-(4-Trifluormethylphenyl)-2-cyano-3-hydroxyhept-2-en-6-in-carbonsäureamid-Lysin- oder Natriumsalz oder 2-Cyano-3-cyclopropyl-3-hydroxy-acryisäure-(4-cyanophenyl)-amid-Lysin- oder Natriumsalz einsetzt.

5. Verwendung gemäß einem oder mehreren der Ansprüche 1 bis 4 zur Herstellung eines Arzneimittels zur Behandlung von Leukämie, chronische Leukämie des T- und B-Zelltyps, Hodgkin's oder non- Hodgkin's Lymphom, Karzinome, Lungenkrebs, Eierstockkrebs, Lymphknotenkrebs, Sarkome, Kaposi's Sarkom, Meningiom, Darmkrebs, Hirntumore, Brustkrebs, Magenkrebs, Pankreaskrebs, Prostatakrebs oder Hautkrebs.

## Claims

1. The use of a sodium or lysinium salt of the compound of the formula II 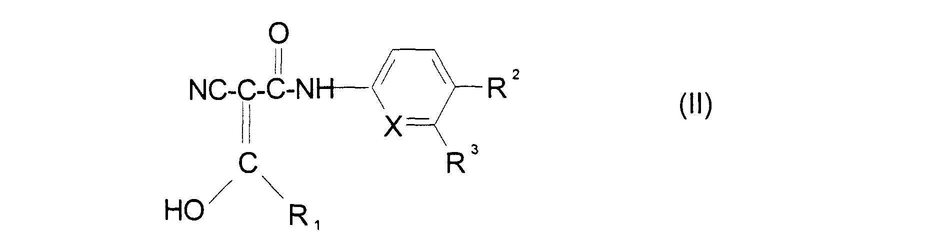 and/or an optionally stereoisomeric form of the sodium or lysinium salt of the compound of the formula II for the production of a pharmaceutical for the treatment of carcinomatous disorders, where
R¹ is
a) (C₃-C₅)-cycloalkyl,
b) (C₂-C₆)-alkenyl or
c) (C₂-C₆)-alkynyl,
R² is
a) -CF₃,
b) -O-CF₃,
c) -S-CF₃,
d) -OH,
e) -NO₂,
f) halogen,
g) benzyl,
h) phenyl,
i) -CN,
k) -O-phenyl or
I) -O-phenyl, mono- or polysubstituted by
1) (C₁-C₄)-alkyl,
2) halogen,
3) -O-CF₃ or
4) -O-CH₃,
R³ is
a) (C₁-C₄)-alkyl,
b) halogen or
c) a hydrogen atom, and
X is
a) a -CH group or
b) a nitrogen atom.

2. The use as claimed in claim 1, where
R¹ is
a) cyclopropyl,
b) (C2-C3)-alkenyl or
c) (C₃-C₅)-alkynyl,
R² is
a) -CF₃,
b) -O-CF₃,
c) -S-CF₃,
d) -O-phenyl,
e) -phenyl,
f) -CN or
g) -O-phenyl, mono- or polysubstituted by (C₁-C₄)-alkyl or halogen,
R³ is a hydrogen atom or methyl, and
X is a -CH group.

3. The use as claimed in claim 1 or 2, where
R¹ is cyclopropyl, C₃-alkenyl or C₄-alkynyl,
R² is -S-CF₃, CN, 2-methyl-4-chlorophenyl or CF₃,
R³ is a hydrogen atom, and
X is a -CH group.

4. The use as claimed in one or more of claims 1 to 3, wherein N-(4-trifluoromethylphenyl)-2-cyano-3-hydroxyhept-2-en-6-ynecarboxamide lysine or sodium salt or 2-cyano-3-cyclopropyl-3-hydroxyacrylic acid (4-cyanophenyl)amide lysine or sodium salt is employed.

5. The use as claimed in one or more of claims 1 to 4 for the production of a pharmaceutical for the treatment of leukemia, chronic leukemia of the T- or B-cell type, Hodgkin's or non-Hodgkin's lymphoma, carcinoma, lung cancer, ovarian cancer, lymph node cancer, sarcoma, Kaposi's sarcoma, meningioma, intestinal cancer, brain tumors, breast cancer, stomach cancer, pancreatic cancer, prostatic cancer or skin cancer.

## Revendications

1. Utilisation d'un sel de sodium ou de lysinium du composé de formule II 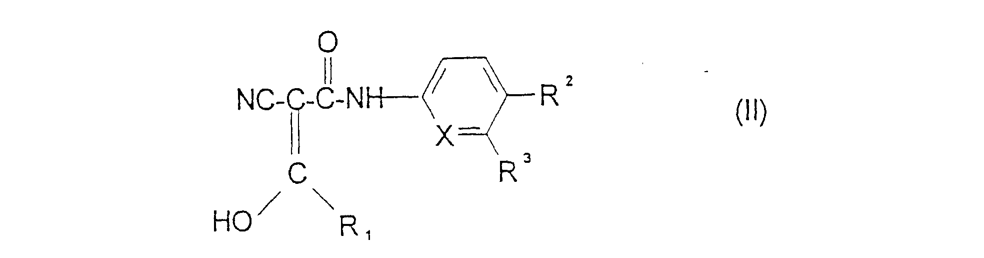 et/ou d'une forme éventuellement stéréoisomère du sel de sodium ou de lysinium du composé de formule II, pour la fabrication d'un médicament destiné au traitement de maladies cancéreuses, formule dans laquelle
R¹ représente
a) un groupe cycloalkyle en C₃-C₅,
b) un groupe alcényle en C₂-C₆ ou
c) un groupe alcynyle en C₂-C₆,
R² représente
a) -CF₃,
b) -O-CF₃,
c) -S-CF₃,
d) -OH,
e) -NO₂,
f) un atome d'halogène,
g) le groupe benzyle,
h) le groupe phényle,
i) -CN,
k) le groupe -O-phényle ou
l) un groupe -O-phényle substitué une ou plusieurs fois par un ou des
1) groupes alkyle en C₁-C₄,
2) atomes d'halogène,
3) -O-CF₃ ou
4) -O-CH₃,
R³ représente
a) un groupe alkyle en C₁-C₄,
b) un atome d'halogène ou
c) un atome d'hydrogène, et
X représente
a) un groupe -CH ou
b) un atome d'azote.

2. Utilisation selon la revendication 1, dans laquelle
R¹ représente
a) le groupe cyclopropyle,
b) un groupe alcényle en C₂-C₃ ou
c) un groupe alcynyle en C₃-C₅,
R² représente
a) -CF₃,
b) -O-CF₃,
c) -S-CF₃,
d) le groupe -O-phényle,
e) le groupe phényle,
f) -CN ou
g) un groupe -O-phényle substitué une ou plusieurs fois par un ou des groupes alkyle en C₁-C₄ ou atomes d'halogène,
R³ représente un atome d'halogène ou le groupe méthyle, et
X représente un groupe -CH.

3. Utilisation selon la revendication 1 ou 2, dans laquelle
R¹ représente un groupe cyclopropyle, alcényle en C₃ ou alcynyle en C₄,
R² représente un groupe -S-CF₃, CN, 2-méthyl-4-chlorophényle ou CF₃,
R³ représente un atome d'hydrogène et
X représente un groupe -CH.

4. Utilisation selon une ou plusieurs des revendications 1 à 3, caractérisée en ce que l'on utilise le sel de sodium ou de lysinium du N-(4-trifluorométhylphényl)-2-cyano-3-hydroxyhept-2-én-6-ynecarboxamide ou le sel de sodium ou de lysinium du 2-cyano-3-cyclopropyl-3-hydroxy-(4-cyanophényl)acrylamide.

5. Utilisation selon une ou plusieurs des revendications 1 à 4, pour la fabrication d'un médicament destiné au traitement de la leucémie, de la leucémie chronique du type à lymphocytes B et T, du lymphome d'Hodgkin ou non hodgkinien, de carcinomes, du cancer du poumon, du cancer de l'ovaire, du cancer des ganglions lymphatiques, de sarcomes, du sarcome de Kaposi, d'un méningiome, du cancer de l'intestin, de tumeurs du cerveau, du cancer du sein, du cancer de l'estomac, du cancer du pancréas, du cancer de la prostate ou du cancer de la peau.
